(19) Europäisches Patentamt
European Patent Office
Office européen des brevets

(11) EP 1 254 650 B1

(12) **EUROPEAN PATENT SPECIFICATION**

(45) Date of publication and mention of the grant of the patent:
**18.07.2007 Bulletin 2007/29**

(51) Int Cl.:
***A61Q 5/10*** *(2006.01)* ***A61K 8/49*** *(2006.01)*
***A61K 8/41*** *(2006.01)*

(21) Application number: **02008285.5**

(22) Date of filing: **22.04.2002**

(54) **Hair dye composition**

Haarfärbemittel

Produit pour la coloration des cheveux

(84) Designated Contracting States:
**DE FR GB**

(30) Priority: **23.04.2001 JP 2001124256**
**23.04.2001 JP 2001124257**
**23.04.2001 JP 2001124258**

(43) Date of publication of application:
**06.11.2002 Bulletin 2002/45**

(73) Proprietor: **KAO CORPORATION**
**Tokyo 103-8210 (JP)**

(72) Inventors:
- **Saito, Yoshinori,**
**Kao Corporation,**
**Res. Lab.**
**Sumida-ku,**
**Tokyo 131-8501 (JP)**
- **Koike, Kenzo,**
**Kao Corporation,**
**Res. Lab.**
**Sumida-ku,**
**Tokyo 131-8501 (JP)**

(74) Representative: **HOFFMANN EITLE**
**Patent- und Rechtsanwälte**
**Arabellastrasse 4**
**81925 München (DE)**

(56) References cited:
**WO-A-99/20234**
**WO-A-99/20236**
**DE-A- 4 208 297**
**DE-A- 19 827 000**
**FR-A- 2 662 701**

EP 1 254 650 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

BACKGROUND OF THE INVENTION

1. Field of the Invention

The present invention relates to one-pack type hair dye compositions capable of dyeing the hair, with excellent dyeing ability, in a desired color shade ranging from a natural chestnut color to a black color without a reddish cast by making use of oxygen in the air.

2. Description of the Related Art

It is known that indoline derivatives or indole derivatives substituted, at each of the 5- and/or 6-position thereof, with a hydroxyl group are converted to a melanin dye by oxygen in the air. This reaction is used for hair dye compositions. For example, in Japanese Patent Publication No. 2996724, use of 5,6-dihydroxyindoline as an oxidation dye for keratinous fibers is proposed and according to the patent, this compound can oxidatively produce color when exposed to oxygen in the air. When 5,6-dihydroxyindoline is used singly as an oxidation dye precursor and ammonia, which is an ordinarily employed alkali agent (and is given as only one example in the literature), is used alone as an alkali agent, however, hair is dyed in a reddish color. This drawback is particularly inappropriate for covering gray or white hair of those who have naturally black hair and in addition, the dyeing ability of the resulting dye composition is not sufficient.

In the above-described patent, described is use of another oxidation dye precursor or a direct dye in combination as a method for improving the shade to be produced by the hair dye using 5,6-dihydroxyindoline. In WO93/09758, use in combination with a natural dye is proposed. In JP3695594 described is addition of a small amount of 5,6-dihydroxyindoline as an additive to a hair dye formulation containing an ordinarily employed oxidation dye or an oxidation dye precursor. In WO99/06016, proposed is use of a coupling component of a dye precursor, preferably 2,4-diaminophenoxyethanol in order to suppress development of the controversial reddish color.

In Japanese Patent Publication No. 2710589, proposed is a method of dyeing the hair in an intense coloring by applying thereto (A) a hair dye composition containing an indole or indoline compound and a manganese salt and (B) a composition containing a basifying agent in two stages.

As described above, however, any of the related arts for improving the shade produced by a hair dye using 5,6-dihydroxyindoline needs combined use with another oxidation dye precursor or a direct dye. A technique of using 5,6-dihydroxyindoline singly as an oxidation dye precursor, thereby producing a color shade appropriate for covering gray or white hair of those having naturally black hair is not known yet.

In the technique disclosed in Japanese Patent No. 2710589, dyeing is conducted in two stages. It therefore involves problems such as necessity of labor and skilled work, for example, by a beautician specialized in dyeing.

WO 99/20236 refers to a ready-to-use oxidation dyeing composition for keratin fibers, and in particular, for human keratin fibers such as hair comprising, in an appropriate dyeing medium, a least an auto-oxidable dye, and at least one oxidoreductase-type enzyme with two electrons in the presence of at least a donor for said enzyme. The auto-oxidable dye can be selected from benzenes, indoles or indolines.

In FR-A-2 662 701 dye compositions for keratin fibers, and especially of human keratin fibers are disclosed, which comprise 5,6-dihydroxyindoline derivatives.

SUMMARY OF THE INVENTION

An object of the present invention is to provide a one-pack type hair dye composition which has excellent dyeing ability, is suited for covering gray or white hair of those having naturally black hair, and permits easy hair dyeing into a color shade ranging from a natural chestnut color to a black color without a reddish cast without using an oxidizing agent harmful for the hair and by using only an indoline compound as a dye precursor.

The present inventors have found that by using an indoline derivative or salt thereof in combination with a specific amine or salt thereof and ammonia and regulating a pH to a specific range, air-oxidative, one-pack type hair dye having excellent dyeing ability and capable of dyeing the hair into a black color without a reddish cast is available ; the coexistence of the amine with ammonia at a proper ratio makes it possible to produce a desired color shade ranging from a natural chestnut color to a black color without a reddish cast.

In the present invention, there is thus provided an air-oxidative, one-pack type hair dye composition comprising an indoline derivative represented by the following formula (1):

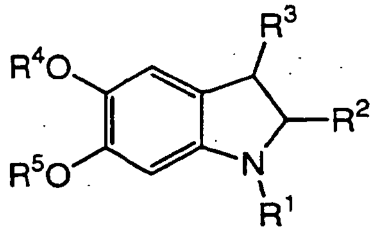

(1)

wherein, $R^1$, $R^4$ and $R^5$ each independently represents a hydrogen atom, a $C_{1-4}$ alkyl group or a $C_{2-20}$ acyl group, and $R^2$ and $R^3$ each independently represents a hydrogen atom or a $C_{1-4}$ alkyl group, or salt thereof, and an amine represented by the following formula (2):

$$H_2N\text{-}CH_2\text{-}R \qquad (2)$$

wherein, R represents a hydrogen atom, a $C_{1-5}$ alkyl group which may be substituted with a hydroxyl group or a phenyl group which may have a substituent, or salt thereof, and ammonia and has a pH ranging from 8.5 to 11.

The hair dye composition of the present invention containing only an indoline derivative as a dye precursor has excellent dyeing ability, is capable of easily dyeing the hair into a color shade ranging from a natural chestnut color to a black color without a reddish cast so that it is particularly suited for covering gray or white hair of those having naturally black hair.

## BRIEF DESCRIPTION OF THE DRAWINGS

FIG. 1 illustrates a difference in dyeing ability, depending on the kind of an alkali agent used in combination with the indoline derivative.

## DETAILED DESCRIPTION OF THE INVENTION

Examples of the indoline derivative (1) usable in the present invention include 5,6-dihydroxyindoline, N-methyl-5,6-dihydroxyindoline, N-ethyl-5,6-dihydroxyindoline, N-butyl-5,6-dihydroxyindoline, 5-methoxy-6-hydroxyindoline, 2-methyl-5,6-dihydroxyindoline, 3-methyl-5,6-dihydroxyindoline, and 5-hydroxy-6-methoxyindoline. Of these, 5,6-dihydroxyindoline is preferred. Examples of the salts of these indoline derivatives include hydrochloride, hydrobromide, sulfate, phosphate, acetate, propionate, lactate and citrate. Of these, hydrobromide is preferred. Most preferred as the indoline derivative (1) or salt thereof is 5,6-dihydroxyindoline hydrobromide. This 5,6-dihydroxyindoline may be formed by incorporating it in the form of 5,6-diacyloxyindoline to a hair dye composition and then hydrolyzing the mixture under basic conditions.

At least two of the above-described indoline derivatives (1) or salts thereof may be used in combination. Its (their) content is preferably 0.01 to 10 wt.%, especially 0.1 to 5 wt.% based on the whole composition (the whole composition except a propellant when the composition is in the aerosol form containing the propellant) in order to dye the hair into a shade ranging from a natural chestnut color to a black color without a reddish cast, which shade is suited for covering gray or white hair of those having naturally black hair.

Examples of the amine (2) usable in the present invention include monoethanolamine, isopropanolamine, butylamine, and benzylamine. Examples of its salt include hydrochloride, hydrobromide, sulfate, phosphate, acetate, propionate, lactate and citrate. Of these amines (2) and salts thereof, monoethanolamine and isopropanolamine are particularly preferred from the viewpoint of odor. At least two of these amines and salts thereof may be used in combination.

When the amine (2) or salt thereof is used alone as an alkali agent, hair can be dyed in a black color without a reddish cast. Use of ammonia in combination as the alkali agent makes it possible to dye the hair in a shade ranging from a natural chestnut color to a black color without a reddish cast. By a dye containing only ammonia as an alkali agent, hair is dyed in a shade inclining to red so such a dye is not effective for covering gray or white hair of those having naturally black hair.

A weight ratio of the amine (2) or salt thereof to ammonia is preferably 1:99 to 100:0, more preferably 15:85 to 100:0, especially 15:85 to 99:1. Within this range, hair can be dyed in a shade ranging from a natural chestnut color to a black color without a reddish cast which shade is suited for covering gray or white hair of those having naturally black hair. The greater the weight ratio of the amine (2) or salt thereof relative to ammonia, the more the shade inclines to

black. The greater the weight ratio of ammonia relative to the amine (2) or salt thereof, on the other hand, the more the shade inclines to chestnut. By adjusting their ratio in the hair dye composition, a desired color shade ranging from chestnut to black is available.

The total content of the alkali agent including the amine (2) or salt thereof and ammonia is preferably 0.01 to 20 wt.%, especially 0.1 to 10 wt.% based on the whole composition. Within this range, the composition has sufficient dyeing ability and is mild to skin.

It is possible to incorporate, in the hair dye composition of the present invention, an antioxidant in order to prevent a deterioration in dyeing ability with the passage of time due to oxidation of the indoline derivative (1) or salt thereof. Preferred examples include sodium sulfite, ascorbic acid, thioglycolic acid and salts thereof, L-cysteine and salts thereof, and N-acetyl-L-cysteine and salts thereof. Sodium sulfite is especially preferred, because it not only contributes to stabilization of a dye precursor but also improves the dyeing ability.

At least two of these antioxidants may be used in combination. Its (their) content is preferably 0.01 to 2 wt.%, especially 0.05 to 1 wt.% based on the whole composition. Within this range, hair can be dyed in a shade ranging from a natural chestnut color to a black color without a reddish cast, which shade is suited for covering gray or white hair of those having naturally black hair.

The hair dye composition of the invention provides light touch when it is not thickened. Use of a thickener, on the other hand, can dispel the fear of sagging without damaging the dyeing ability. The viscosity of the hair dye composition (the composition not added with a propellant in the case of an aerosol) is preferably 50 to 50000 $mm^2/s$. Examples of the thickener include cellulose derivatives such as hydroxyethyl cellulose, hydroxyethylcellulose-glycidyl trimethyl ammonium chloride ether, methyl cellulose and carboxymethyl cellulose; natural gums such as xanthan gum and guar gum; and synthetic polymers such as polyvinylpyrrolidone, crosslinked polyacrylic acid and salts thereof, polyacrylic acid and salts thereof and polyacrylamide.

The indoline derivative (1) or salt thereof can be converted into a melanin dye, when reacted with oxygen in the air under basic conditions. By regulating the pH of the hair dye composition of the present invention to 8.5 to 11, preferably 9 to 10.5 when the composition contains the amine (2) or salt thereof as an alkali agent, the composition has improved dyeing ability and moreover, can dye the hair in a shade ranging from a natural chestnut color to a black color without a reddish cast which shade is effective for covering gray or white hair of those having naturally black hair.

As a pH adjusting agent, as well as an essential ingredient such as the amine (2) or salt thereof, or ammonia, ordinarily employed pH regulators such as inorganic or organic basic compounds such as sodium hydroxide, potassium hydroxide, sodium carbonate, sodium bicarbonate, arginine, lysine, histidine and guanidine; and inorganic or organic acids such as hydrochloric acid, sulfuric acid, phosphoric acid, citric acid and lactic acid are usable as needed.

The hair dye composition of the present invention can be prepared in various forms such as cream, gel, lotion and foam. Of these, aerosol is preferred, because this form improves the dyeing ability of the composition and even after repeated use, it does not deteriorate the dyeing ability. The hair dye composition of the present invention in the aerosol form can be prepared by filling a pressure container (aerosol can or the like) with a propellant together with the composition except the propellant.

As the propellant, compressed gas and liquefied gas which are ordinarily employed for aerosol products are usable. Examples of the compressed gas include nitrogen gas, carbon dioxide gas, and argon gas, while those of the liquefied gas include liquefied petroleum gas, saturated lower hydrocarbons, and dimethyl ether. At least two of these propellants may be used in combination. It is (or they are) incorporated in the aerosol type hair dye composition (including it or them) preferably in an amount of 1 to 20 wt.%, especially 3 to 15 wt.% in order to attain a sufficient jet rate. In addition, the internal pressure of an aerosol can filled with the composition is preferably adjusted to 3 to 5 $kg/cm^2G$ (25°C).

It is preferred that upon filling the composition, crimping and deaeration are carried out simultaneously to reduce the air remaining inside the container. Such deaeration is effective for stabilizing the composition in the container. For example, deaeration under pressure not greater than 48 kPa is preferred.

The hair dye composition of the invention in the aerosol form preferably contains a nonionic surfactant as a foaming agent and/or homogenizing agent. Examples of the nonionic surfactant include polyoxyethylene alkyl ethers such as polyoxyethylene oleyl ether and polyoxyethylene stearyl ether; polyoxyethylene alkyl phenyl ethers such as polyoxyethylene nonylphenyl ether and polyoxyethylene octylphenyl ether; polyoxyethylene sorbitan fatty acid esters, fatty acid alkylolamides, and polyoxyethylene-sec-tetradecyl ether. The nonionic surfactant is preferably added in an amount of 0.01 to 30 wt.%, especially 0.1 to 10 wt.%, based on the whole composition except the propellant.

To the hair dye composition of the invention, it is possible to add components, which are ordinarily employed for hair dyes, such as surfactant other than the above-described ones, stabilizer, buffer, perfume, touch improver, chelating agent, solubilizing agent, and antiseptic as needed, depending on the purpose.

For hair dyeing with the hair dye composition of the invention, it may be applied to hair, followed by oxidation of the indoline derivative (1) or salt thereof by oxygen in the air. The hair dye composition of the invention is also usable as a gradual hair dye. For example, after application of it to gray or white hair, the hair is allowed to stand for 1 to 10 minutes. When this operation is repeated twice or several times, the gray or white hair can be covered gradually without

being noticed by someone. The hair dye composition of the invention can be used at room temperature, but its dyeing ability can be improved by feeding the dyed hair with heat and oxygen by a drier.

Examples

Example 1 - Comparison in dyeing ability among dye solutions containing different alkali agents -

Dye solutions were prepared by mixing 1 wt.% of 5,6-dihydroxyindoline hydrobromide and, as an alkali agent, ammonia, monoethanolamine, isopropanolamine, 2-amino-2-methylpropanol, sodium carbonate and potassium carbonate, respectively, so as to adjust the concentration of each of the alkali agent to 0.1, 0.25, 0.5, 1 or 2 mol/kg. To the tress of goat hair, 1 g of the dye solution was applied. After dyeing at 30°C for 15 minutes, the tress was washed with water, shampooed, rinsed and then dried.

Its dyeing ability was evaluated by color difference ($\Delta E$). The color of the dyed tress was measured by a spectrophotometer ("CM-2002", manufactured by Minolta Co., Ltd.) and $\Delta E$ was calculated in accordance with the below-described equation. The pH and $\Delta E$ of each dye solution was plotted in FIG. 1. In FIG. 1, "MEA", "iPrOHA" and "AMPOH" mean monoethanolamine, isopropanolamine and 2-amino-2-methylpropanol, respectively.

$$\Delta E = \{(L_1-L_0)^2 + (a_1-a_0)^2 + (b_1-b_0)^2\}^{1/2}$$

($L_0$, $a_0$, $b_0$) : color value of goat hair tress before dyeing
($L_1$, $a_1$,$b_1$) : color value of goat hair tress after dyeing

Comparative Examples 19 to 21 and Comparative Examples 1 to 8 - Comparison in the shade of dyed hair among dye solutions containing different alkali agents -

Dye solutions having the compositions as shown in Tables 1 to 3 were prepared and applied to gray or white hair. After dyeing at 30°C for 15 minutes, the hair was washed with water, shampooed, rinsed and then dried.

The shade of the dyed hair was evaluated in accordance with the following standards.

(Evaluation Standards)

A: very dark gray
B: dark gray
C: light gray
D: pale gray with a reddish cast

Table 1

| | | | (wt.%) |
|---|---|---|---|
| | Comparative Examples | | |
| | 19 | 20 | 21 |
| 5,6-Dihydroxyindoline hydrobromide | 1 | 1 | 1 |
| Monoethanolamine | 3.1 | - | - |
| Isopropanolamine | - | 3.8 | - |
| Benzylamine | - | - | 5.4 |
| Water | Balance | Balance | Balance |
| pH | 10.3 | 10.4 | 10.5 |
| Shade of dyed hair | A | A | A |

Table 2

| (wt.%) | | | | |
|---|---|---|---|---|
| | Comparative Examples | | | |
| | 1 | 2 | 3 | 4 |
| 5,6-Dihydroxyindoline hydrobromide | 1 | 1 | 1 | 1 |
| 2-Amino-2-methylpropanol | 4.5 | - | - | - |
| 2-Amino-2-methyl-1,3-propanediol | - | 5.3 | - | - |
| Morpholine | - | - | 4.4 | |
| Guanidine carbonate | - | - | - | 4.5 |
| Water | Balance | Balance | Balance | Balance |
| pH | 10.5 | 9.7 | 9.5 | 10.1 |
| Shade of dyed hair | B | B | B | B |

Table 3

| (wt.%) | | | | |
|---|---|---|---|---|
| | Comparative Examples | | | |
| | 5 | 6 | 7 | 8 |
| 5,6-Dihydroxyindoline hydrobromide | 1 | 1 | 1 | 1 |
| 28 wt.% aqueous ammonia | 3 | - | - | - |
| Sodium carbonate | - | 2.7 | - | - |
| Potassium carbonate | - | - | 3.5 | |
| Arginine | - | - | - | 8.7 |
| Water | Balance | Balance | Balance | Balance |
| pH | 10.1 | 10.1 | 10.2 | 10.0 |
| Shade of dyed hair | D | C | C | C |

Comparative Examples 22 to 24 and Comparative Examples 9 to 13 - pH dependence of dyeing ability -

Dye solutions having the compositions as shown in Tables 4 and 5 were prepared and in a similar manner and similar standards to those employed for Comparative Examples 19 to 21 and Comparative Examples 1 to 8, the shade of the dyed hair was evaluated.

Table 4

| (wt.%) | | | | |
|---|---|---|---|---|
| | Comp. Ex. | Comparative Examples | | |
| | 9 | 22 | 23 | 24 |
| 5,6-Dihydroxyindoline hydrobromide | 1 | 1 | 1 | 1 |
| Monoethanolamine | 3 | 3 | 3 | 3 |
| 10 wt.% Sulfuric acid | q.s. for pH regulation | q.s. for pH regulation | q.s. for pH regulation | q.s. for pH regulation |
| Water | Balance | Balance | Balance | Balance |
| pH | 8 | 8.5 | 9 | 10 |
| Shade of dyed hair | C | B | A | A |

Table 5

| (wt.%) | | | | |
|---|---|---|---|---|
| | Comparative Examples | | | |
| | 10 | 11 | 12 | 13 |
| 5,6-Dihydroxyindoline hydrobromide<br>28 wt.% Aqueous ammonia<br>10 wt.% Sulfuric acid<br><br>Water | 1<br>3<br>q.s. for pH regulation<br>Balance | 1<br>3<br>q.s. for pH regulation<br>Balance | 1<br>3<br>q.s. for pH regulation<br>Balance | 1<br>3<br>q.s. for pH regulation<br>Balance |
| pH | 8 | 8.5 | 9 | 10 |
| Shade of dyed hair | D | D | D | D |

Comparative Example 25 and Comparative Example 14 - Test on repeated dyeing -

Dye solutions having the compositions as shown in Table 6 were prepared and applied to gray or white hair. After dyeing at 30°C for 15 minutes, the hair was washed with water, shampooed, rinsed and then dried. A series of these operations was repeated five times and the shade of the dyed hair was evaluated by the following standards:

(Evaluation standards)

A: very dark gray
B: dark gray with a reddish cast

Table 6

| (wt.%) | | |
|---|---|---|
| | Comp.Ex.25 | Comp. Ex. 14 |
| 5,6-Dihydroxyindoline hydrobromide<br>Monoethanolamine<br>28 wt.% aqueous ammonia<br>Water | 0.1<br>0.3<br>-<br>Balance | 0.1<br>-<br>0.3<br>Balance |
| pH | 10.3 | 10.0 |
| Shade of hair dyed 5 times | B | D |

Examples 9 to 11, Comparative Examples 15 - Combined use of amine and ammonia -

Gels having the compositions as shown in Table 7 were prepared and applied to gray or white hair. After dyeing at 30°C for 15 minutes, the hair was washed with water, shampooed, rinsed and then dried. The shade of the hair thus dyed with each of the gels is also shown in Table 7.

Table 7

| (wt.%) | | | | |
|---|---|---|---|---|
| | Examples | | | Comp. Ex. |
| | 9 | 10 | 11 | 15 |
| 5,6-Dihydroxyindoline hydrobromide<br>Monoethanolamine | 1<br>2.7 | 1<br>1.5 | 1<br>0.6 | 1<br>- |

(continued)

| (wt.%) | | | | |
|---|---|---|---|---|
| | Examples | | | Comp. Ex. |
| | 9 | 10 | 11 | 15 |
| 28 wt.% Aqueous ammonia | 0.3 | 1.5 | 2.4 | 3.0 |
| Sodium sulfite | 0.1 | 0.1 | 0.1 | 0.1 |
| Xanthan gum | 1 | 1 | 1 | 1 |
| Water | Balance | Balance | Balance | Balance |
| pH | 10.3 | 10.2 | 10.1 | 10.0 |
| Shade of dyed hair | Black | Dark chestnut | Chestnut | Chestnut strongly tinged with red |

Examples 12 to 14, Comparative Example 16 - Combined use of amine and ammonia -

Gels having the compositions as shown in Table 8 were prepared. A series of operations including dyeing, washing and drying were repeated 5 times. The shade of the hair thus dyed 5 times with each of the gels is also shown in Table 8.

Table 8

| (wt.%) | | | | |
|---|---|---|---|---|
| | Examples | | | Comp. Ex. |
| | 12 | 13 | 14 | 16 |
| 5,6-Dihydroxyindoline hydrobromide | 0.1 | 0.1 | 0.1 | 0.1 |
| Monoethanolamine | 0.27 | 0.15 | 0.06 | - |
| 28 wt.% Aqueous ammonia | 0.03 | 0.15 | 0.24 | 0.3 |
| Sodium sulfite | 0.1 | 0.1 | 0.1 | 0.1 |
| Xanthan gum | 1 | | 1 | 1 |
| Water | Balance | Balance | Balance | Balance |
| pH | 10.3 | 10.2 | 10.1 | 10.0 |
| Shade of hair dyed 5 times | Black | Dark chestnut | Chestnut | Chestnut strongly tinged with red |

Comparative Examples 26 and 27, and Comparative Examples 17 and 18 - Comparison in dyeing ability among dye compositions having different forms -

An aerosol container was filled with, as a stock solution, each of the compositions (viscosity: 18000 $mm^2/s$) of Comparative Examples 26 and 27 as shown in Table 9 together with a propellant (LPG) (stock solution : propellant = 90:10, weight ratio).

To 1 g of the tress of gray or white hair, 1 g (in terms of a stock solution in the case of an aerosol type hair dye) of each of aerosol type hair dyes of Comparative Examples 26 and 27 and creamy hair dyes of Comparative Examples 17 and 18 was applied. After dyeing at 30°C for 15 minutes, the hair was washed with water, shampooed, rinsed and then dried. These operations were repeated at 5 times in total at intervals of 7 days. The color difference (ΔE) before and after dyeing was determined as in Example 1 and is shown in Table 9.

Table 9

| Composition (wt.%) | Comp. Ex. (aerosol) | | Comp. Ex. (creamy), | |
|---|---|---|---|---|
| | 26 | 27 | 17 | 18 |
| 5,6-Dihydroxyindoline hydrobromide | 0:35 | | 0.35 | |
| Softanol 90 | 0.1 | | - | |
| Monoethanolamine | 3 | | - | |

(continued)

| Composition (wt.%) | Comp. Ex. (aerosol) | | Comp. Ex. (creamy), | |
|---|---|---|---|---|
| | 26 | 27 | 17 | 18 |
| Monoethanolamine hydrochloride | Amount to adjust pH to 9 | | - | |
| Xantan gum | 1 | | - | |
| Cetyl/stearyl alcohol (30:70) | - | | 6 | |
| Coconut oil fatty alcohol | - | | 2 | |
| Cetyl/stearyl alcohol (30:70) polyglycol ether (20EO) | - | | 2 | |
| 28wt.% aqueous ammonia | - | | 3 | |
| Ammonium sulfate | - | | Amount to adjust pH to 9 | |
| Sodium sulfite | 0.1 | - | 0.1 | - |
| Ascorbic acid | - | 0.1 | - | 0.1 |
| Water | Balance | | Balance | |
| Color difference (ΔE) before and after dyeing | 45 | 43 | 35 | 35 |

Example 17 - Dyeing method by feeding the dyed hair with heat and oxygen by a drier -

To 1 g of the tress of gray or white hair, 1 g (in terms of a stock solution) of the aerosol type hair dye of comparative Example 26 was applied. After hot air was sent to the hair by a drier for 15 minutes, the hair was washed with water, shampooed, rinsed and then dried. A series of these operations were repeated 5 times in total at intervals of 7 days. The color difference (ΔE) before and after dyeing was found to be 48.

## Claims

1. An air-oxidative, one-pack type hair dye composition comprising an indoline derivative represented by the following formula (1) :

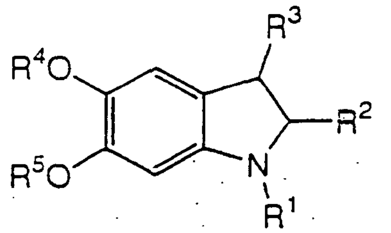

(1)

wherein, $R^1$, $R^4$ and $R^5$ each independently represents a hydrogen atom, a $C_{1-4}$ alkyl group or a $C_{2-20}$ acyl group, and $R^2$ and $R^3$ each independently represents a hydrogen atom or a $C_{1-4}$ alkyl group, or salt thereof ; an amine represented by the following formula (2):

$$H_2N\text{-}CH_2\text{-}R \qquad (2)$$

wherein, R represents a hydrogen atom, a $C_{1-5}$ alkyl group which may be substituted with a hydroxyl group or a phenyl group which may have a substituent, or salt thereof; and ammonia, and has a pH ranging from 8.5 to 11.

2. A one-pack type hair dye composition in the aerosol form, which comprises a stock solution containing a composition of Claim 1 and an antioxidant ; and a propellant.

3. A hair dyeing method, which comprises applying a one-pack type hair dye composition of any one of Claims 1 or 2

to the hair.

**4.** A hair dyeing method, which comprises applying a one-pack type hair dye composition of any one of Claims 1 or 2 to the hair and feeding the dyed hair with heat and oxygen by a drier.

**5.** A hair dyeing method of Claim 3 or 4, further comprising adjusting a weight ratio, in the hair dye composition, of the amine of the formula (2) or salt thereof to ammonia, whereby the hair is dyed in a desired shade ranging from chestnut to black.

## Patentansprüche

**1.** Luftoxidative einteilige Haarfärbezusammensetzung, umfassend ein Indolinderivat mit der folgenden Formel (1):

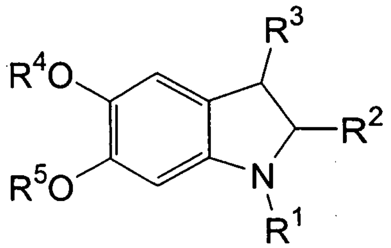

(1)

worin $R^1$, $R^4$ und $R^5$ jeweils unabhängig ein Wasserstoffatom, eine $C_{1-4}$-Alkylgruppe oder eine $C_{2-20}$-Acylgruppe sind und $R^2$ und $R^3$ jeweils unabhängig ein Wasserstoffatom oder eine $C_{1-4}$-Alkylgruppe sind, oder ein Salz davon; ein Amin mit der folgenden Formel (2):

$$H_2N\text{-}CH_2\text{-}R \qquad (2)$$

worin R ein Wasserstoffatom, eine $C_{1-5}$-Alkylgruppe, die durch eine Hydroxyl- oder Phenylgruppe substituiert sein kann, die einen Substituenten haben kann, oder ein Salz davon ist, und Ammoniak und mit einem pH im Bereich von 8,5 bis 11.

**2.** Einteilige Haarfärbezusammensetzung in Aerosolform, umfassend eine Grundlösung umfassend eine Zusammensetzung nach Anspruch 1 und ein Antioxidans und ein Treibmittel.

**3.** Haarfärbeverfahren, umfassend das Auftragen einer einteiligen Haarfärbezusammensetzung nach einem der Ansprüche 1 oder 2 auf das Haar.

**4.** Haarfärbeverfahren, umfassend das Auftragen einer einteiligen Haarfärbezusammensetzung nach einem der Ansprüche 1 oder 2 auf das Haar und die Zufuhr von Wärme und Sauerstoff durch einen Trockner zum gefärbten Haar.

**5.** Haarfärbeverfahren nach Anspruch 3 oder 4, weiterhin umfassend das Einstellen eines Gewichtsverhältnisses des Amins der Formel (2) oder des Salzes davon zu Ammoniak in der Haarfärbezusammensetzung, wodurch das Haar zu einer gewünschten Schattierung im Bereich von Kastanie bis Schwarz gefärbt wird.

## Revendications

**1.** Composition de colorant capillaire de type une seule solution, s'oxydant à l'air, comprenant un dérivé indoline représenté par la formule (1) suivante :

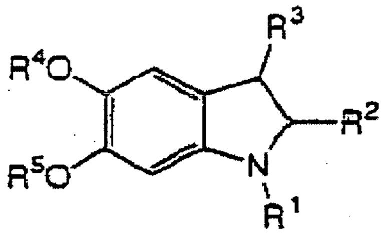

(1)

dans laquelle, $R^1$, $R^4$ et $R^5$ représentent chacun indépendamment un atome d'hydrogène, un groupe alkyle en $C_1$-4 ou un groupe acyle en $C_{2-20}$, et $R^2$ et $R^3$ représentent chacun indépendamment un atome d'hydrogène ou un groupe alkyle en $C_{1-4}$, ou un sel de celui-ci ; une amine représentée par la formule (2) suivante:

$H_2N$-$CH_2$-R (2)

dans laquelle, R représente un atome d'hydrogène, un groupe alkyle en Ci-5 qui peut être substitué par un groupe hydroxyle ou un groupe phényle qui peut avoir un substituant, ou un sel de celle-ci ; et de l'ammoniaque, et a un pH variant de 8,5 à 11.

**2.** Composition de colorant capillaire de type une seule solution sous forme d'aérosol, qui comprend une solution mère contenant une composition selon la revendication 1 et un antioxydant ; et un agent propulseur.

**3.** Procédé de coloration capillaire, qui comprend l'application d'une composition de colorant capillaire de type une seule solution selon l'une quelconque des revendications 1 ou 2 aux cheveux.

**4.** Procédé de coloration capillaire, qui comprend l'application d'une composition de colorant capillaire de type une seule solution selon l'une quelconque des revendications 1 ou 2 aux cheveux colorés et l'alimentation des cheveux en chaleur et oxygène par un séchoir.

**5.** Procédé de coloration capillaire selon la revendication 3 ou 4, comprenant en outre l' ajustement d' un rapport pondéral, dans la composition de colorant capillaire, de l'amine de formule (2) ou du sel de celle-ci à l'ammoniaque, au moyen de quoi les cheveux sont colorés dans une nuance souhaitée variant du châtain au noir.

FIG. 1

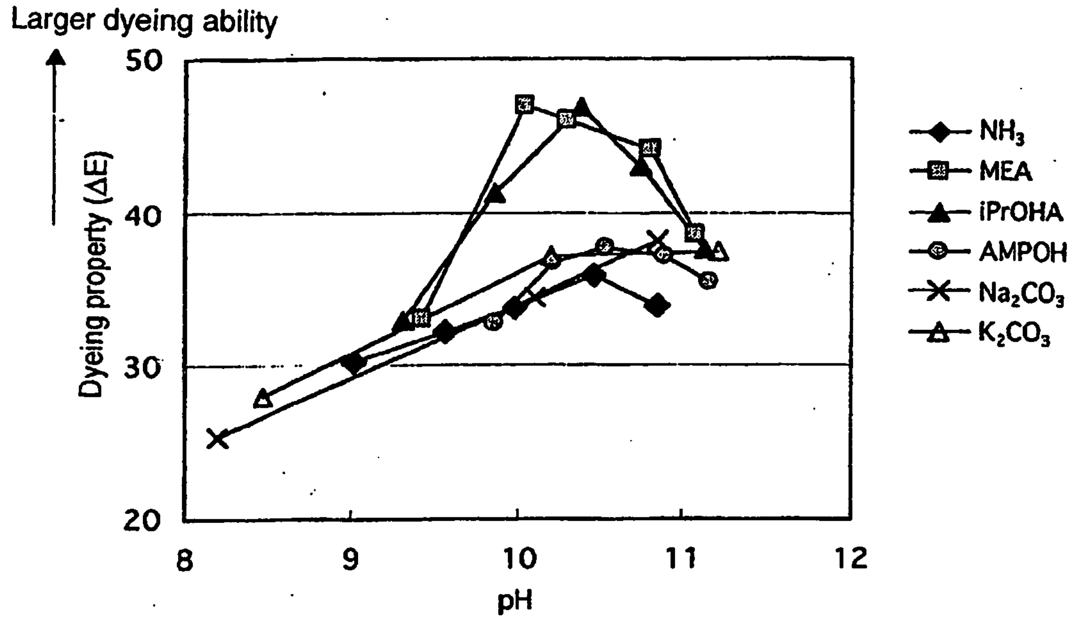
Larger dyeing ability
Dyeing property (ΔE)
50
40
30
20
8
9
10
11
12
pH
NH3
MEA
iPrOHA
AMPOH
Na2CO3
K2CO3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2996724 B
- WO 9309758 A
- JP 3695594 B
- WO 9906016 A
- JP 2710589 B
- WO 9920236 A
- FR 2662701 A